# EUROPEAN PATENT APPLICATION

(11) **EP 4 501 361 A1**
(43) Date of publication of application: **05.02.2025**
(21) Application number: 23780185.7
(22) Date of filing: 24.03.2023
(51) Int. Cl.: A61K 47/22, A61K 9/51, A61K 31/7105, A61K 47/14, A61K 47/24, A61K 47/28, A61K 48/00, A61P 43/00

(54) **LIPID NANOPARTICLES FOR DELIVERING NUCLEIC ACID TO PERIPHERAL BLOOD MONONUCLEAR CELLS, AND METHOD FOR DELIVERING NUCLEIC ACID TO PERIPHERAL BLOOD MONONUCLEAR CELLS USING SAME**

(30) Priority: 28.03.2022 JP 2022051919
(71) Applicant: NOF Corporation, Shibuya-ku Tokyo 150-6019 (JP); National University Corporation Chiba University, Chiba-shi, Chiba 263-8522 (JP)
(72) Inventor: NAKAI, Yuta, Kawasaki-shi, Kanagawa 210-0865 (JP); TANGE, Kota, Kawasaki-shi, Kanagawa 210-0865 (JP); AKITA, Hidetaka, Chiba-shi, Chiba 260-8675 (JP); TANAKA, Hiroki, Chiba-shi, Chiba 260-8675 (JP); SAKURAI, Yu, Chiba-shi, Chiba 260-8675 (JP); MIYAMA, Ryo, Chiba-shi, Chiba 260-8675 (JP)
(74) Representative: Weickmann & Weickmann PartmbB
(86) International application number: PCT/JP2023/011848
(87) International publication number: WO 2023/190175

(57) **Abstract**

The present invention provides lipid nanoparticles for delivering nucleic acid to peripheral blood mononuclear cells that can improve the efficiency of nucleic acid delivery to peripheral blood mononuclear cells, and a method for delivering nucleic acid to peripheral blood mononuclear cells by using same. Lipid nanoparticles for use in delivering a nucleic acid to a peripheral blood mononuclear cell, containing an ionic lipid represented by the formula (1), a phospholipid that is 1,2-diacyl-sn-glycero-3-phosphocholine in which the acyl groups have not less than 20 carbon atoms and at least one of the acyl groups is an alkenoyl group, cholesterol, and a dimyristoylglycerol PEG represented by the formula: CH₂(OR⁶)-CH(OR⁷)-CH₂(OR⁸) (symbols in the formula are as described in the specification), and a method for delivering a nucleic acids to a peripheral blood mononuclear cell by using same.

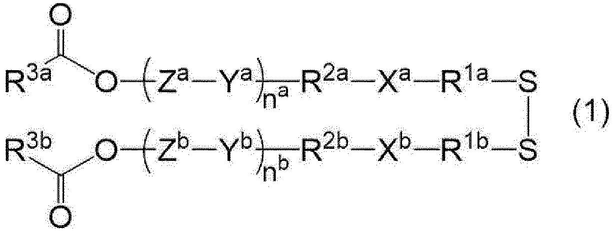

## Description

### [Technical Field]

The present invention relates to lipid nanoparticles for use in delivering nucleic acid to peripheral blood mononuclear cells, and a method for delivering nucleic acid to peripheral blood mononuclear cells by using same.

### [Background Art]

For practicalization of nucleic acid therapy using oligonucleic acids such as siRNA, and gene therapy using mRNA, pDNA, and the like, an effective and safe nucleic acid delivery carrier is demanded. While virus vectors are nucleic acid delivery carriers with good expression efficiency, the development of non-viral nucleic acid delivery carriers that can be used more safely is ongoing.

In in vitro nucleic acid delivery, an electroporation method is being developed in addition to viral vectors. The electroporation method involves physically introducing nucleic acids by opening pores in cells, and therefore, cellular stress and cytotoxicity pose problems. On the other hand, lipid nanoparticles using ionic lipids are being developed as low-toxicity nucleic acid delivery carriers that can be used both in vitro and in vivo.

Ionic lipids are broadly constituted of an amine moiety and a lipid moiety. For example, in ionic lipids, the amine moiety that protonates under acidic conditions and the polyanionic nucleic acid interact electrostatically to form lipid nanoparticles, which in turn promotes cellular uptake and delivers the nucleic acid into cells.

A known ionic lipid that is widely used in general is 1,2-dioleoyloxy-3-dimethylaminopropane (DODAP). It is known that by combining such known ionic lipid with phospholipid, cholesterol, and PEG lipid, lipid nanoparticles can be formed and nucleic acids can be delivered into cells (see Non Patent Literature 1).

For example, Patent Literature 1 describes an ionic lipid having a structure in which compounds consisting of one or two amine moieties and one lipid moiety are connected by a biodegradable disulfide bond. Patent Literature 1 shows that the ionic lipid improves pharmacokinetics such as blood stability, tumor targeting performance and the like. In addition, it has been shown that, by changing the structure around the amine moiety, pKa of the lipid membrane structure can be adjusted to a value favorable for endosomal escape within the cell, and that the cleavage of disulfide bonds within the cell can be used to dissociate nucleic acids from the lipid membrane structure. In fact, since the ionic lipid shows a higher nucleic acid delivery efficiency than the known ionic lipid DODAP, it has been revealed that the ionic lipid can improve intracellular dynamics, such as improved delivery efficiency of nucleic acids into the cytoplasm.

For example, Patent Literature 2 shows a lipid membrane structure that uses an ionic lipid that has an aromatic ring introduced near the lipid moiety in addition to a tertiary amine moiety and disulfide bond, thereby improving the ability to fuse with the endosomal membrane and further increasing the efficiency of nucleic acid delivery to the cytoplasm.

As described above, ionic lipids that improve intracellular dynamics by increasing endosomal escape efficiency and membrane fusion ability have been developed, and it has been shown that nucleic acids can be delivered efficiently to established cell lines and living organisms.

### [Citation List]

### [Patent Literature]

[Patent Literature 1]
   US 2014/0335157 A
[Patent Literature 2]
   WO 2019/188867
[Patent Literature 3]
   WO 2020/039631

### [Non Patent Literature]

[Non Patent Literature 1]
   Molecular Therapy, 25(7):1467-1475 (2017)
[Non Patent Literature 2]
   Molecular Therapy, 26(6): 1509-1519 (2018)
[Non Patent Literature 3]
   Adv. Funct. Mater., 30: 1910575 (2020)
[Non Patent Literature 4]
   Gene Therapy, 23: 699-707 (2016)
[Non Patent Literature 5]
   Nanomedicine: NBM, 13: 1377-1387 (2017)

### [Summary of Invention]

### [Technical Problem]

Incidentally, efficient nucleic acid delivery to blood cells such as peripheral blood mononuclear cells is important in the field of cancer immunotherapy, such as CAR-T therapy, and regenerative medicine, such as the creation and differentiation of iPS cells. However, the efficiency of nucleic acid delivery to peripheral blood mononuclear cells, such as T cells extracted from the body, is insufficient with lipid nanoparticles made of ionic lipids, and development using viral vectors and electroporation methods is still performed preferentially.

Therefore, in order to introduce nucleic acids more efficiently by lipid nanoparticles, further improvement in the efficiency of nucleic acid delivery to peripheral blood mononuclear cells is required. For example, Patent Literature 3 shows that the efficiency of nucleic acid delivery to peripheral blood mononuclear cells can be improved by making the surface of lipid nanoparticles cationic under physiological pH conditions.

However, since compounds that are cationic under physiological pH conditions generally have high cytotoxicity, it is desirable to use neutral lipid nanoparticles with low cytotoxicity for practical use.

Non Patent Literature 2, Non Patent Literature 3 and Non Patent Literature 4 each show the use of ionic lipids with different structural features as the lipid nanoparticles for liver delivery. In these documents, different phospholipids are used as components of lipid nanoparticles, and the optimal combination with phospholipids varies depending on the structure of the ionic lipids.

On the other hand, Non Patent Literature 5 shows an example in which the appropriate phospholipid varies depending on the cell type to be transfected, even for lipid nanoparticles using the same ionic lipid.

As mentioned above, there are examples where the efficiency of nucleic acid introduction into peripheral blood mononuclear cells has been improved using lipid nanoparticles. However, because the appropriate phospholipid varies depending on the structure of the ionic lipid, the improvement is not fully satisfactory for practical use, and further improvements in nucleic acid delivery efficiency are required.

In view of the above-mentioned problems, the present invention aims to provide lipid nanoparticles for delivering nucleic acid to peripheral blood mononuclear cells that can improve the efficiency of nucleic acid delivery to peripheral blood mononuclear cells, and a method for delivering nucleic acid to peripheral blood mononuclear cells by using same.

### [Solution to Problem]

As described above, the appropriate combination of ionic lipid and phospholipid in lipid nanoparticles varies depending on the cell type, and efficient nucleic acid delivery requires optimizing the combination of ionic lipid and phospholipid.

The present inventors have conducted intensive studies in view of the above-mentioned problems and found that neutral lipid nanoparticles produced using an ionic lipid that has a pKa suitable for endosomal escape and that decomposes specifically in the reductive environment within the cell, and a specific 1,2-diacyl-sn-glycero-3-phosphocholine as the phospholipid, can improve the efficiency of nucleic acid delivery to peripheral blood mononuclear cells. The present invention based on this finding provides the following.
[1] A lipid nanoparticle for use in delivering a nucleic acid to a peripheral blood mononuclear cell, comprising an ionic lipid represented by the formula (1):

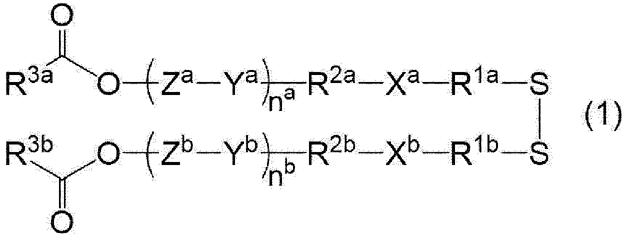

   (in the formula (1),
   R^{1a} and R^{1b} are each independently an alkylene group having 1 - 6 carbon atoms,
   X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
   R^{2a} and R^{2b} are each independently an alkylene group or an oxydialkylene group each having not more than 8 carbon atoms,
   Y^{a} and Y^{b} are each independently an ester bond, an amide bond, a carbamate bond, an ether bond or a urea bond,
   Z^{a} and Z^{b} are each independently a divalent group derived from an aromatic compound having 3 - 16 carbon atoms and at least one aromatic ring, and optionally having a hetero atom,
   n^{a} and n^{b} are each independently 0 or 1, and
   R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):

      R⁹-O-CO-(CH₂)a- (4)

      (in the formula (4),
      R⁹ is an aliphatic hydrocarbon group having 2 - 20 carbon atoms, and
      a is an integer of 2 to 10)),
   a phospholipid that is 1,2-diacyl-sn-glycero-3-phosphocholine in which the acyl groups have not less than 20 carbon atoms and at least one of the acyl groups is an alkenoyl group, cholesterol, and
   a dimyristoylglycerol PEG represented by the formula (2):

      CH₂(OR⁶)-CH(OR⁷)-CH₂(OR⁸) (2)

      (in the formula (2), any two of R⁶, R⁷ and R⁸ are myristoyl groups, and the remaining one is an alkyl group having 1 - 6 carbon atoms connected via a polyethylene glycol chain having a number average molecular weight of 1,000 to 3,000).
[2] The lipid nanoparticle of [1], wherein the aforementioned phospholipid is at least one selected from the group consisting of 1,2-dieicosenoyl-sn-glycero-3-phosphocholine (DEiPC), 1,2-dierucoyl-sn-glycero-3-phosphocholine (DEPC), and 1,2-dinervonoyl-sn-glycero-3-phosphocholine (DNPC).
[3] The lipid nanoparticle of [1] or [2], wherein the ionic lipid represented by the formula (1) is an ionic lipid represented by the following formula: 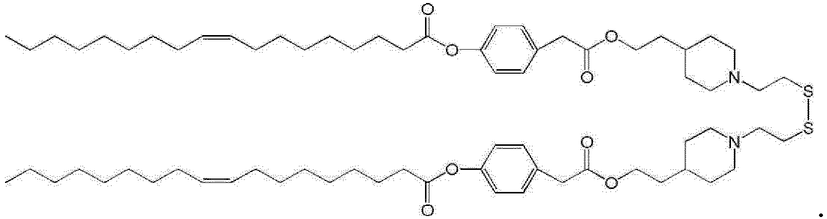
[4] The lipid nanoparticle of any one of [1] to [3], wherein the aforementioned ionic lipid is 20 to 60 mol%, the aforementioned phospholipid is 5 to 20 mol%, the aforementioned cholesterol is 30 to 70 mol%, and the aforementioned dimyristoylglycerol PEG lipid is 0.5 to 1.5 mol% relative to the total of the aforementioned ionic lipid, the aforementioned phospholipid and the aforementioned cholesterol.
[5] A method for delivering a nucleic acid to a peripheral blood mononuclear cell, comprising contacting the lipid nanoparticle of any one of [1] to [4] encapsulating the nucleic acid with the peripheral blood mononuclear cell.
[6] Use of a lipid nanoparticle comprising an ionic lipid represented by the formula (1):

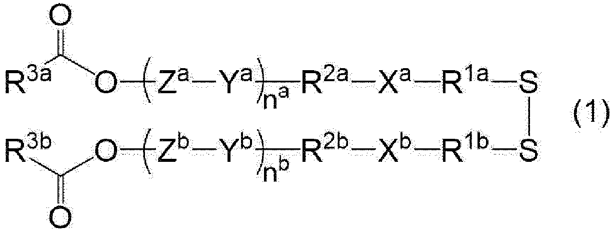

   (in the formula (1), each symbol is as defined in [1]),
   a phospholipid that is 1,2-diacyl-sn-glycero-3-phosphocholine in which the acyl groups have not less than 20 carbon atoms and at least one of the acyl groups is an alkenoyl group, cholesterol, and
   a dimyristoylglycerol PEG represented by the formula (2):

      CH₂(OR⁶)-CH(OR⁷)-CH₂(OR⁸) (2)

      (in the formula (2), each symbol is as defined in [1]),
      in the production of a medicament for delivering a nucleic acid to a peripheral blood mononuclear cell.
[7] The use of [6], wherein the aforementioned phospholipid is at least one selected from the group consisting of 1,2-dieicosenoyl-sn-glycero-3-phosphocholine (DEiPC), 1,2-dierucoyl-sn-glycero-3-phosphocholine (DEPC), and 1,2-dinervonoyl-sn-glycero-3-phosphocholine (DNPC).
[8] The use of [6] or [7], wherein the ionic lipid represented by the formula (1) is an ionic lipid represented by the following formula: 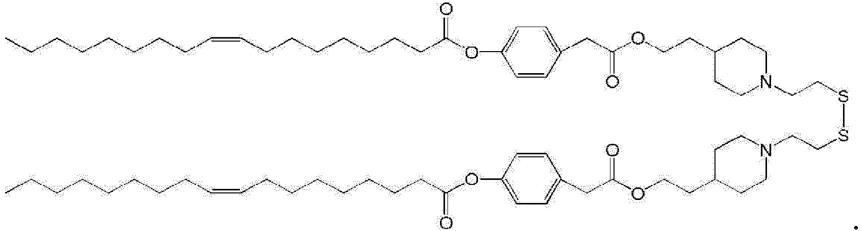
[9] The use of any one of [6] to [8], wherein the aforementioned ionic lipid is 20 to 60 mol%, the aforementioned phospholipid is 5 to 20 mol%, the aforementioned cholesterol is 30 to 70 mol%, and the aforementioned dimyristoylglycerol PEG lipid is 0.5 to 1.5 mol% relative to the total of the aforementioned ionic lipid, the aforementioned phospholipid and the aforementioned cholesterol.

### [Advantageous Effects of Invention]

The lipid nanoparticles of the present invention can efficiently deliver nucleic acids to peripheral blood mononuclear cells by optimizing the combination of ionic lipids and phospholipids and the lipid composition.

### [Brief Description of Drawings]

[Fig. 1]

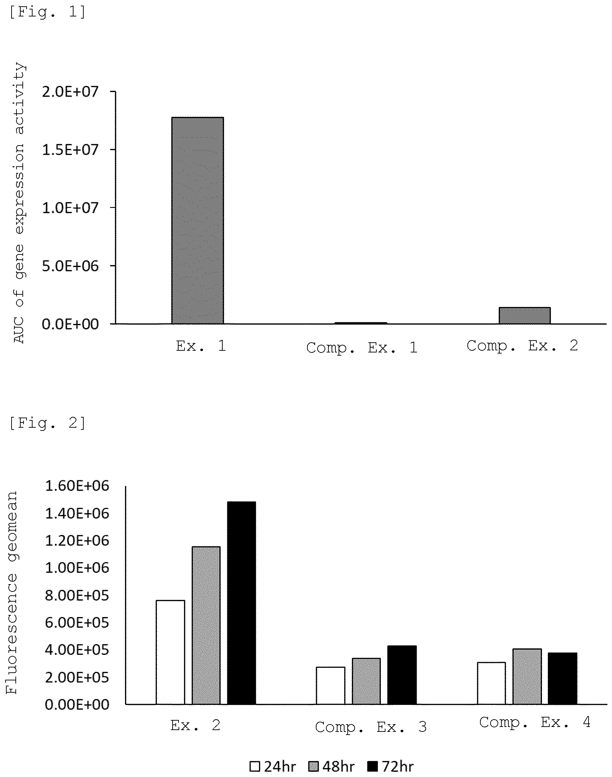

   Fig. 1 is a graph showing the gene expression activity of lipid nanoparticles of Example 1, Comparative Example 1, and Comparative Example 2 in mouse primary T cells.
[Fig. 2]
   Fig. 2 is a graph showing the amount of uptake of lipid nanoparticles of Example 2, Comparative Example 3, and Comparative Example 4 into mouse primary T cells.
[Fig. 3]

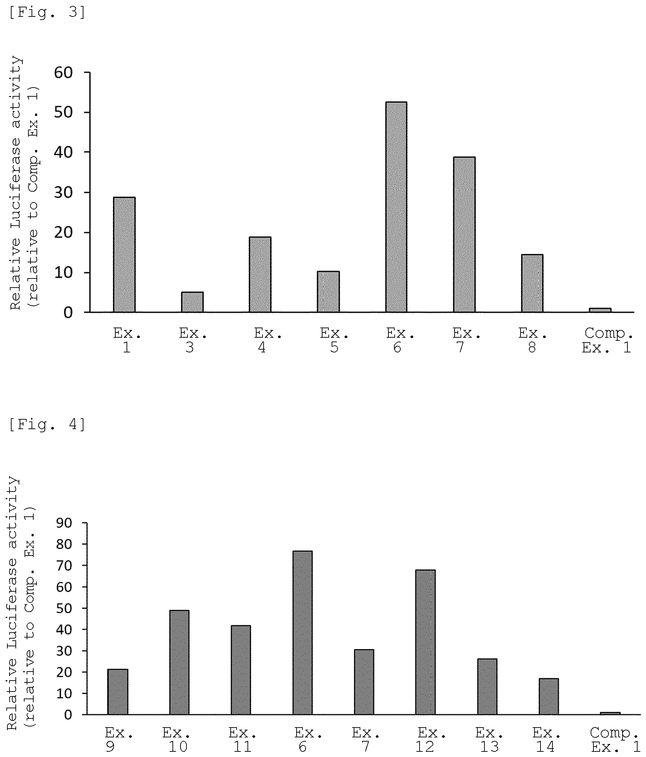

   Fig. 3 is a graph showing the gene expression activity of lipid nanoparticles of Examples 1 and 3 to 8, and Comparative Example 1 in mouse primary T cells.
[Fig. 4]
   Fig. 4 is a graph showing the gene expression activity of lipid nanoparticles of Examples 6, 7 and 9 to 14, and Comparative Example 1 in mouse primary T cells.
[Fig. 5]

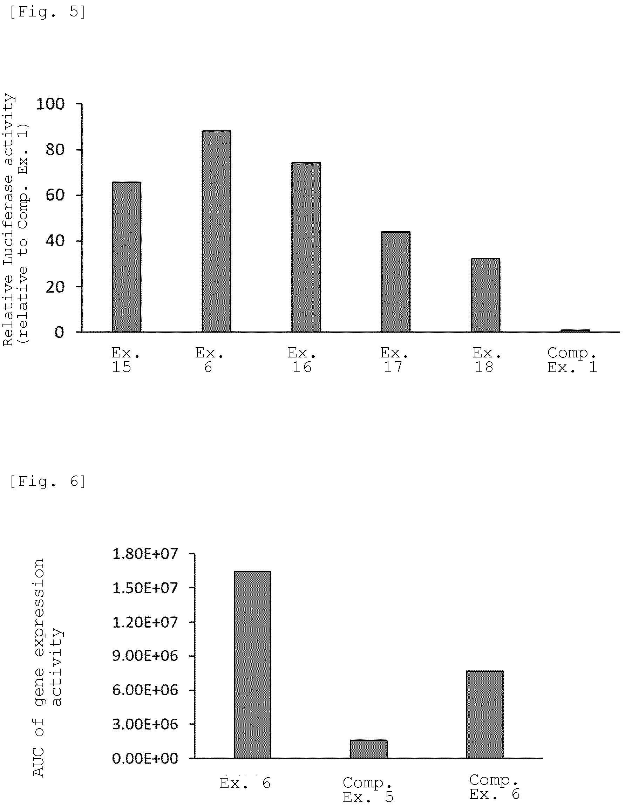

   Fig. 5 is a graph showing the gene expression activity of lipid nanoparticles of Examples 6 and 15 to 18, and Comparative Example 1 in mouse primary T cells.
[Fig. 6]
   Fig. 6 is a graph showing the gene expression activity of lipid nanoparticles of Example 6, Comparative Example 5, and Comparative Example 6 in human primary T cells.

### [Description of Embodiments]

While the embodiments of the present invention are explained in the following, the present invention is not limited thereto. The present invention relates to lipid nanoparticles containing an ionic lipid represented by the formula (1) (i.e., ionic lipid having a tertiary amino group, a lipid moiety, and a disulfide bond which is a biodegradable group), a phospholipid that is 1,2-diacyl-sn-glycero-3-phosphocholine in which the acyl groups have not less than 20 carbon atoms and at least one of the acyl groups is an alkenoyl group, cholesterol, and a dimyristoylglycerol PEG represented by the formula (2), and a method for delivering nucleic acids to peripheral blood mononuclear cells.

### Lipid nanoparticles

In the present specification, the "lipid nanoparticle" (sometimes to be abbreviated as "LNP" in the present specification) means a particle having a membrane structure wherein the hydrophilic groups of amphiphilic lipid are arranged in the interface, facing the aqueous phase side and having a particle size of less than 1 um, and the "amphiphilic lipid" means a lipid having both a hydrophilic group and a hydrophobic group.

The particle size of the lipid nanoparticles of the present invention is preferably 10 nm to 500 nm, more preferably 30 nm to 300 nm. The particle size can be measured by using a particle size distribution measuring device such as Zetasizer Nano (Malvern) or the like. The particle size of the lipid nanoparticles can be appropriately adjusted by the method for producing the lipid nanoparticles. In the present specification, the "particle size" means an average particle size (Zeta mean) measured by a dynamic light scattering method.

Examples of the amphiphilic lipid include ionic lipid, phospholipid, PEG lipid, and the like. In the present specification, "PEG" means polyethylene glycol, "PEG lipid" means lipid modified with PEG, and "Y modified with X" (e.g., X:PEG, Y:lipid) means Y to which X is bonded. In other words, "PEG lipid" means lipid to which PEG is bonded.

The lipid nanoparticles of the present invention may contain lipids other than ionic lipid represented by the formula (1), phospholipid, cholesterol, and dimyristoylglycerol PEG represented by the formula (2) (hereinafter sometimes referred to as "other lipids"). Examples of other lipids include sterols other than cholesterol and PEG lipids other than dimyristoylglycerol PEG represented by the formula (2).

The amount of other lipids in the lipid nanoparticles of the present invention is preferably 0 to 50 mol%, more preferably 0 to 30 mol%, further preferably 0 to 10 mol%, relative to the total amount of lipids in the lipid nanoparticles. As used herein, the total amount of lipids in the lipid nanoparticles means, for example, when the lipid nanoparticles contain an ionic lipid represented by the formula (1), a phospholipid, cholesterol, a dimyristoylglycerol PEG represented by the formula (2), and other lipids as constituents, the "total amount of ionic lipid represented by the formula (1), phospholipid, cholesterol, dimyristoylglycerol PEG represented by the formula (2), and other lipids". In the present specification, the "amount of B relative to A (mol%)" means "100 x amount of B (mol)/amount of A (mol)". For example, the "amount of other lipids relative to the total amount of lipids (mol%)" means "100 x amount of other lipids (mol)/total amount of lipids (mol)".

In the present invention, no use of other lipids is most preferred, namely, the lipids constituting the lipid nanoparticles of the present invention most preferably consist of an ionic lipid represented by the formula (1), phospholipid, cholesterol, and a dimyristoylglycerol PEG represented by the formula (2).

### Ionic lipid

The ionic lipid used in the present invention is an ionic lipid represented by the following formula (1) (sometimes to be abbreviated as "ionic lipid (1)" in the present specification). Only one kind of ionic lipid (1) may be used, or two or more kinds thereof may be used in combination.

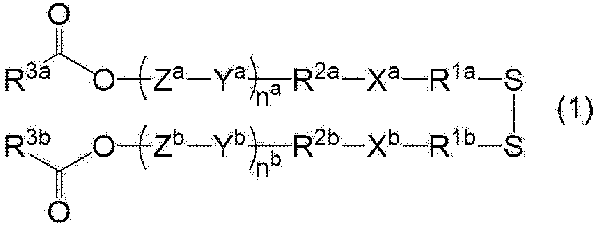

(in the formula (1),
R^{1a} and R^{1b} are each independently an alkylene group having 1 - 6 carbon atoms,
X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 - 16 carbon atoms and at least one aromatic ring, and optionally having a hetero atom,
n^{a} and n^{b} are each independently 0 or 1, and
R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):

   R⁹-O-CO-(CH₂) a- (4)

   (in the formula (4),
   R⁹ is an aliphatic hydrocarbon group having 2 - 20 carbon atoms, and a is an integer of 2 to 10)).
   R^{1a} and R^{1b} are each independently an alkylene group having 1 - 6 carbon atoms, and may be linear or branched, preferably linear. The carbon number of the alkylene group is preferably 1 - 4, more preferably 1 - 2. Specific examples of the alkylene group having 1 - 6 carbon atoms include methylene group, ethylene group, trimethylene group, isopropylene group, tetramethylene group, isobutylene group, pentamethylene group, neopentylene group and the like. Preferably, R^{1a} and R^{1b} are each independently a methylene group, an ethylene group, a trimethylene group, an isopropylene group or a tetramethylene group, most preferably an ethylene group.
   R^{1a} may be the same as or different from R^{1b}, and R^{1a} is preferably the same group as R^{1b}.
   X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups, preferably each independently a cyclic alkylene tertiary amino group having 2 - 5 carbon atoms and 1 - 2 tertiary amino groups.

The alkyl group having 1 - 6 carbon atoms in the non-cyclic alkyl tertiary amino group having 1 - 6 carbon atoms and one tertiary amino group may be linear, branched or cyclic. The alkyl group preferably has a carbon number of 1 to 3. Specific examples of the alkyl group having 1 - 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like, preferably methyl group, ethyl group, propyl group or isopropyl group, most preferably methyl group.

A preferable specific structure of the non-cyclic alkyl tertiary amino group having 1 - 6 carbon atoms and one tertiary amino group is represented by X¹.

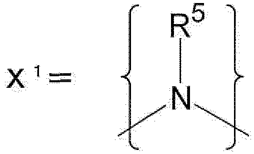

R⁵ in X¹ is an alkyl group having 1 - 6 carbon atoms, which may be linear, branched or cyclic. The alkyl group preferably has a carbon number of 1 - 3. Specific examples of the alkyl group having 1 - 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like. R⁵ is preferably methyl group, ethyl group, propyl group or isopropyl group, most preferably methyl group.

The carbon number of the cyclic alkylene tertiary amino group having 2 - 5 carbon atoms and 1 - 2 tertiary amino groups is preferably 4 - 5. The cyclic alkylene tertiary amino group having 2 - 5 carbon atoms and 1 - 2 tertiary amino groups is specifically aziridylene group, azetidylene group, pyrrolidylene group, piperidylene group, imidazolidylene group, or piperazylene group, preferably pyrrolidylene group, piperidylene group, or piperazylene group, most preferably piperidylene group.

A preferable specific structure of the cyclic alkylene tertiary amino group having 2 - 5 carbon atoms and one tertiary amino group is represented by X².

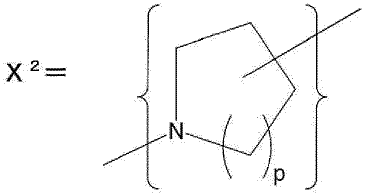

The p of X² is 1 or 2. When p is 1, X² is a pyrrolidylene group, and when p is 2, X² is a piperidylene group. Preferably, p is 2.

A preferable specific structure of the cyclic alkylene tertiary amino group having 2 - 5 carbon atoms and two tertiary amino groups is represented by X³.

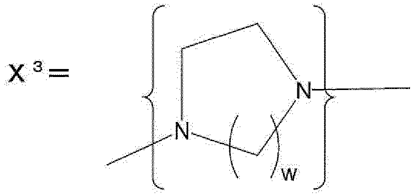

The w of X³ is 1 or 2. When w is 1, X³ is an imidazolidylene group, and when w is 2, X³ is a piperazylene group.

X^{a} may be the same as or different from X^{b}, and X^{a} is preferably the same group as X^{b}.

R^{2a} and R^{2b} are each independently an alkylene group or an oxydialkylene group each having not more than 8 carbon atoms, preferably each independently an alkylene group having not more than 8 carbon atoms.

The alkylene group having not more than 8 carbon atoms may be linear or branched, preferably linear. The number of carbons contained in the alkylene group is preferably not more than 6, most preferably not more than 4. Specific examples of the alkylene group having not more than 8 carbon atoms include methylene group, ethylene group, trimethylene group, isopropylene group, tetramethylene group, isobutylene group, pentamethylene group, hexamethylene group, heptamethylene group, octamethylene group and the like, preferably methylene group, ethylene group, trimethylene group, and tetramethylene group, most preferably ethylene group.

In the present specification, the "oxydialkylene group having not more than 8 carbon atoms" means a group containing alkylene groups via an ether bond (alkylene-O-alkylene, in other words, "alkyleneoxyalkylene group"), wherein the total carbon number of the two alkylene groups present is 8 or below. The two alkylene groups present may be the same or different, preferably the same. Specific examples of the oxydialkylene group having not more than 8 carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group) and the like. Preferably, it is oxydimethylene group, oxydiethylene group, oxydi(trimethylene) group, most preferably oxydiethylene group.

R^{2a} may be the same as or different from R^{2b}, and R^{2a} is preferably the same group as R^{2b}.

Y^{a} and Y^{b} are each independently an ester bond, an amide bond, a carbamate bond, an ether bond or a urea bond, preferably each independently an ester bond, an amide bond or a carbamate bond, more preferably each independently an ester bond or an amide bond, most preferably each an ester bond. The direction of the bond of Y^{a} and Y^{b} is not limited. When Y^{a} and Y^{b} are ester bonds, the structure of -Z^{a}-CO-O-R^{2a}- or -Z^{b}-CO-OR^{2b}- is preferably shown.

Y^{a} may be the same as or different from Y^{b}, and Y^{a} is preferably the same group as Y^{b}.

Z^{a} and Z^{b} are each independently a divalent group derived from an aromatic compound having 3 - 16 carbon atoms and at least one aromatic ring, and optionally having a hetero atom. The number of carbons contained in the aromatic compound is preferably 6 to 12, most preferably 6 or 7. The aromatic ring contained in the aromatic compound is preferably one.

As the kind of the aromatic ring contained in the aromatic compound having 3 - 16 carbon atoms, benzene ring, naphthalene ring, and anthracene ring can be mentioned for aromatic hydrocarbocycle, and imidazole ring, pyrazole ring, oxazole ring, isoxazole ring, thiazole ring, isothiazole ring, triazine ring, pyrrole ring, furanthiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxazine ring, phenothiazine ring, acridine ring and the like can be mentioned for aromatic heterocycle. It is preferably benzene ring, naphthalene ring, or anthracene ring, most preferably benzene ring.

The aromatic ring may have a substituent. Examples of the substituent include acyl group having 2 - 4 carbon atoms, alkoxycarbonyl group having 2 - 4 carbon atoms, carbamoyl group having 2 - 4 carbon atoms, acyloxy group having 2 - 4 carbon atoms, acylamino group having 2 - 4 carbon atoms, alkoxycarbonylamino group having 2 - 4 carbon atoms, fluorine atom, chlorine atom, bromine atom, iodine atom, alkylsulfanyl group having 1 - 4 carbon atoms, alkylsulfonyl group having 1 - 4 carbon atoms, arylsulfonyl group having 6 - 10 carbon atoms, nitro group, trifluoromethyl group, cyano group, alkyl group having 1 - 4 carbon atoms, ureido group having 1 - 4 carbon atoms, alkoxy group having 1 - 4 carbon atoms, aryl group having 6 - 10 carbon atoms, aryloxy group having 6 - 10 carbon atoms, and the like. Preferable examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group and the like.

A preferable specific structure of Z^{a} and Z^{b} is Z¹.

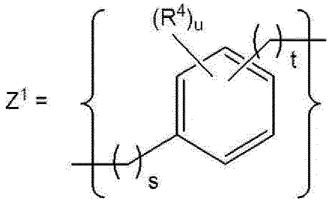

wherein s is an integer of 0 - 3, t is an integer of 0 - 3, u is an integer of 0 - 4, and R⁴ in the number of u are each independently a substituent.

The s for Z¹ is preferably an integer of 0 or 1, more preferably 0.

The t for Z¹ is preferably an integer of 0 to 2, more preferably 1.

The u for Z¹ is preferably an integer of 0 to 2, more preferably an integer of 0 or 1.

The R⁴ for Z¹ is a substituent of an aromatic ring (benzene ring) contained in the aromatic compound having 3 - 16 carbon atoms which does not inhibit the reaction in the synthesis process of an ionic lipid. Examples of the substituent include acyl group having 2 - 4 carbon atoms, alkoxycarbonyl group having 2 - 4 carbon atoms, carbamoyl group having 2 - 4 carbon atoms, acyloxy group having 2 - 4 carbon atoms, acylamino group having 2 - 4 carbon atoms, alkoxycarbonylamino group having 2 - 4 carbon atoms, fluorine atom, chlorine atom, bromine atom, iodine atom, alkylsulfanyl group having 1 - 4 carbon atoms, alkylsulfonyl group having 1 - 4 carbon atoms, arylsulfonyl group having 6 - 10 carbon atoms, nitro group, trifluoromethyl group, cyano group, alkyl group having 1 - 4 carbon atoms, ureido group having 1 - 4 carbon atoms, alkoxy group having 1 - 4 carbon atoms, aryl group having 6 - 10 carbon atoms, aryloxy group having 6 - 10 carbon atoms, and the like. Preferable examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group and the like. When R⁴ is present in plurality, each R⁴ may be the same or different.

Z^{a} may be the same as or different from Z^{b}, and Z^{a} is preferably the same group as Z^{b}.

n^{a} and n^{b} are each independently 0 or 1.

n^{a} may be the same as or different from n^{b}, and n^{a} is preferably the same as n^{b}.

R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):

R⁹-O-CO-(CH₂)a- (4)

(in the formula (4),
R⁹ is an aliphatic hydrocarbon group having 2 - 20 carbon atoms, and
a is an integer of 2 to 10), preferably each independently a residue derived from a reaction product of a liposoluble vitamin having a hydroxyl group, and succinic anhydride or glutaric anhydride, or an aliphatic hydrocarbon group having 12 - 22 carbon atoms, most preferably each independently an aliphatic hydrocarbon group having 12 - 22 carbon atoms.

The residue derived from a reaction product of a liposoluble vitamin having a hydroxyl group, and succinic anhydride or glutaric anhydride is a group having a structure in which the hydroxyl group of a liposoluble vitamin having a hydroxyl group is replaced by *-O-CO-CH₂-CH₂- or *-O-CO-CH₂-CH₂-CH₂-. * is the bonding position with the liposoluble vitamin. The residue derived from a reaction product of a sterol derivative having a hydroxyl group, and succinic anhydride or glutaric anhydride is a group having a structure in which the hydroxyl group of a sterol derivative having a hydroxyl group is replaced by *-O-CO-CH₂-CH₂- or *-O-CO-CH₂-CH₂-CH₂-. * is the bonding position with the sterol derivative.

The liposoluble vitamin having a hydroxyl group is, for example, retinol, ergosterol, 7-dehydrocholesterol, calciferol, cholecalciferol, dihydroergocalciferol, dihydrotachysterol, tocopherol, tocotrienol and the like. Preferred example of the liposoluble vitamin having a hydroxyl group is tocopherol.

Examples of the sterol derivative having a hydroxyl group include cholesterol, cholestanol, stigmasterol, β-sitosterol, lanosterol, and ergosterol and the like, preferably cholesterol or cholestanol.

The aliphatic hydrocarbon group having 1 - 40 carbon atoms may be linear or branched. The aliphatic hydrocarbon group may be saturated or unsaturated. In the case of an unsaturated aliphatic hydrocarbon group, the aliphatic hydrocarbon group generally contains 1 - 6, preferably 1 - 3, more preferably 1 - 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 12 - 22, more preferably 13 - 19, most preferably 13 - 17. While the aliphatic hydrocarbon group includes an alkyl group, an alkenyl group, an alkynyl group and the like, it is preferably an alkyl group or an alkenyl group. Specific examples of the aliphatic hydrocarbon group having 1 - 40 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, tetracontyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group and the like. The aliphatic hydrocarbon group having 1 - 40 carbon atoms is preferably tridecyl group, pentadecyl group, heptadecyl group, nonadecyl group, heptadecenyl group, heptadecadienyl group, or 1-hexylnonyl group, particularly preferably tridecyl group, heptadecyl group, heptadecenyl group, or heptadecadienyl group.

In one embodiment of the present invention, the aliphatic hydrocarbon group having 1 - 40 (preferably 12 - 22) carbon atoms for R^{3a} or R^{3b} is derived from fatty acid. In this case, the carbonyl carbon derived from fatty acid is contained in - CO-O- in the formula (1). A specific example of the aliphatic hydrocarbon group is a heptadecadienyl group when linoleic acid is used as the fatty acid, and heptadecenyl group when oleic acid is used as the fatty acid.

The alkyl group having a cyclopropane ring and having 3 - 40 carbon atoms for R^{3a} or R^{3b} means an alkyl group having at least one cyclopropane ring in the alkyl chain and having 3 - 40 carbon atoms. The number of carbon atoms in the alkyl group is 3 to 40, and does not include the number of carbon atoms in the cyclopropane ring. The number of cyclopropane rings in the alkyl group is preferably one. The alkyl group having a cyclopropane ring and having 3 - 40 carbon atoms for R^{3a} or R^{3b} is preferably a group represented by the formula (5):

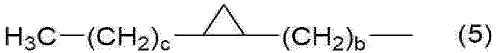

(in the formula (5), b and c are each independently an integer and the total of b and c is 2 to 39). Preferably, b is an integer of 1 to 20, c is an integer of 1 to 19. b is more preferably an integer of 2 to 18, further preferably an integer of 3 to 17, further more preferably an integer of 4 to 12. c is more preferably an integer of 3 to 15, further preferably an integer of 3 to 11, further more preferably an integer of 3 to 9. Examples of the group represented by the formula (5) include 7-(2-octylcyclopropyl)heptyl and the like.

In the formula (4), the aliphatic hydrocarbon group having 2 - 20 carbon atoms for R⁹ may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 or 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 8 to 20, more preferably 9 to 19, further preferably 13 to 19, most preferably 13 to 17. While the aliphatic hydrocarbon group includes an alkyl group, an alkenyl group, an alkynyl group and the like, it is preferably an alkyl group or an alkenyl group, more preferably alkyl group. Specific examples of the aliphatic hydrocarbon group having 2 to 20 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, dodecenyl group, tridecenyl group, tetradecenyl group, pentadecenyl group, hexadecenyl group, heptadecenyl group, octadecyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, icosatrienyl group, icosatetraenyl group, icosapentaenyl group, isostearyl group, 1-hexylheptyl group, 1-ethylnonyl group, 1-butylnonyl group, 1-hexylnonyl group, 1-octylnonyl group, 1-octylundecyl group, 3-octylundecyl group, and the like. The aliphatic hydrocarbon group having 2 - 20 carbon atoms is preferably tridecyl group, pentadecyl group, heptadecyl group, nonadecyl group, heptadecenyl group, heptadecadienyl group, or 1-hexylnonyl group, particularly preferably tridecyl group, heptadecyl group, heptadecenyl group, or heptadecadienyl group.

In the formula (4), a is preferably an integer of 3 to 9, more preferably an integer of 3 to 7, further preferably an integer of 5 to 7, and most preferably 5 or 7.

R^{3a} may be the same as or different from R^{3b}, and R^{3a} is preferably the same group as R^{3b}.

In one embodiment of the present invention, R^{1a} is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

Preferable examples of ionic lipid (1) include the following ionic lipids.

### [Ionic lipid (1-1)]

Ionic lipid (1) wherein
R^{1a} and R^{1b} are each independently an alkylene group having 1 - 6 carbon atoms (e.g., methylene group, ethylene group);
X^{a} and X^{b} are each independently a non-cyclic alkyl tertiary amino group having 1 - 6 carbon atoms and one tertiary amino group (e.g., -N(CH₃)-), or a cyclic alkylene tertiary amino group having 2 - 5 carbon atoms and 1 - 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 - 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride or glutaric anhydride, or an aliphatic hydrocarbon group having 12 - 22 carbon atoms (e.g., heptadecenyl group, heptadecadienyl group, 1-hexylnonyl group).

### [Ionic lipid (1-2)]

Ionic lipid (1) wherein
R^{1a} and R^{1b} are each independently an alkylene group having 1 - 6 carbon atoms (e.g., methylene group, ethylene group);
X^{a} and X^{b} are each independently a non-cyclic alkyl tertiary amino group having 1 - 6 carbon atoms and one tertiary amino group (e.g., -N(CH₃)-), or a cyclic alkylene tertiary amino group having 2 - 5 carbon atoms and 1 - 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 - 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride or glutaric anhydride, or an aliphatic hydrocarbon group having 12 - 22 carbon atoms (e.g., heptadecenyl group, heptadecadienyl group, 1-hexylnonyl group).

### [Ionic lipid (1-3)]

Ionic lipid (1) wherein
R^{1a} and R^{1b} are each independently an alkylene group having 1 - 2 carbon atoms (i.e., methylene group or ethylene group);
X^{a} and X^{b} are each independently X¹:

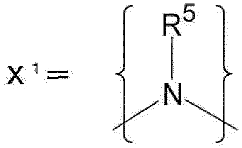

   wherein R⁵ is an alkyl group having 1 - 3 carbon atoms (e.g., methyl group), or X²:

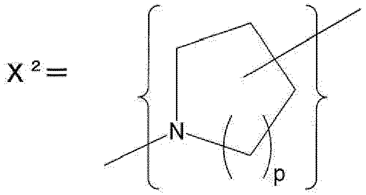

   wherein p is 1 or 2;
   R^{2a} and R^{2b} are each independently alkylene group having not more than 4 carbon atoms (e.g., methylene group, ethylene group, trimethylene group);
   Y^{a} and Y^{b} are each independently an ester bond or an amide bond;
   Z^{a} and Z^{b} are each independently Z¹: 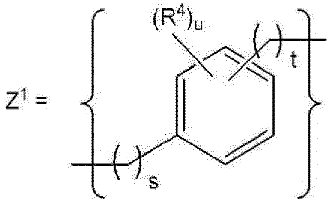
      wherein s is an integer of 0 - 1, t is an integer of 0 - 2, u is an integer of 0 - 2 (preferably 0), and R⁴ in the number of u are each independently a substituent; and
      n^{a} and n^{b} are each independently 0 or 1; and
      R^{3a} and R^{3b} are each independently a residue derived from a reaction product of a liposoluble vitamin having a hydroxyl group (e.g., tocopherol) and succinic anhydride, or an aliphatic hydrocarbon group having 13 - 17 carbon atoms (e.g., heptadecenyl group, heptadecadienyl group, 1-hexylnonyl group).

Specific examples of the ionic lipid (1) include the following O-Ph-P3C1, O-Ph-P4C1, O-Ph-P4C2, O-Bn-P4C2, E-Ph-P4C2, L-Ph-P4C2, HD-Ph-P4C2, O-Ph-amide-P4C2, O-Ph-C3M, and TS-P4C2.

Specific examples of ionic lipid (1) are Lipid 1 to Lipid 20 described in WO2021/195529A2. Particularly, the following Lipid 1, Lipid 5, and Lipid 8 can be mentioned.

**[Table 1-1]**

| name of ionic lipid | structure |
|---|---|
| O-Ph-P3C1 | 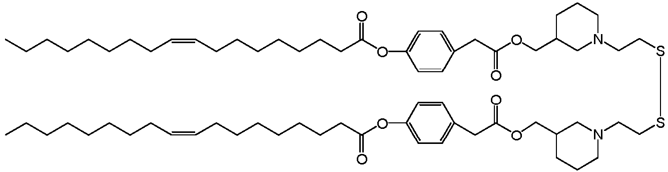 |
| O-Ph-P4C1 | 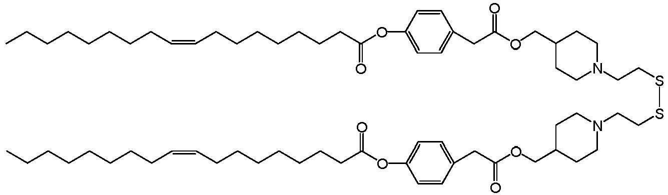 |
| O-Ph-P4C2 (or SS-OP) | 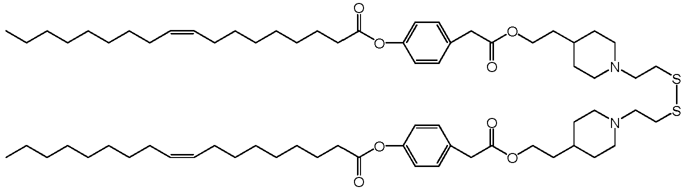 |
| O-Bn-P4C2 | 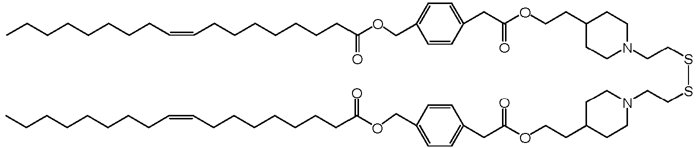 |
| E-Ph-P4C2 (or SS-EP) | 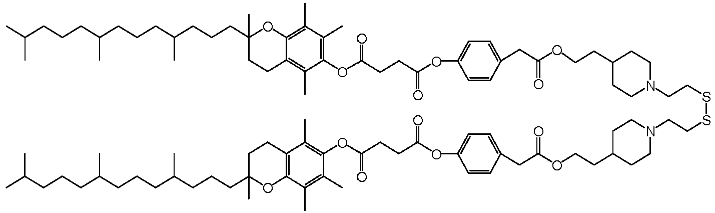 |

**[Table 1-2]**

| name of ionic lipid | structure |
|---|---|
| L-Ph-P4C2 | 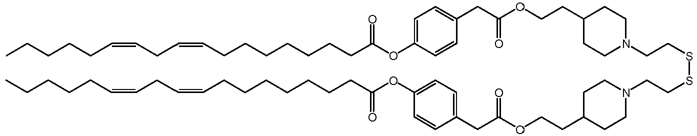 |
| HD-Ph-P4C2 | 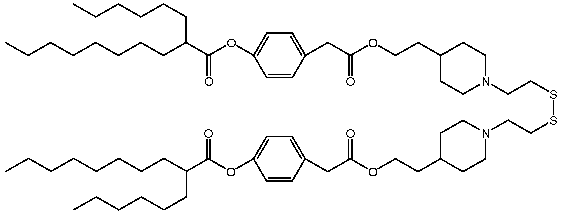 |
| O-Ph-amide-P4C2 | 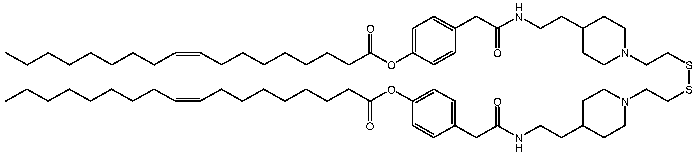 |
| O-Ph-C3M | 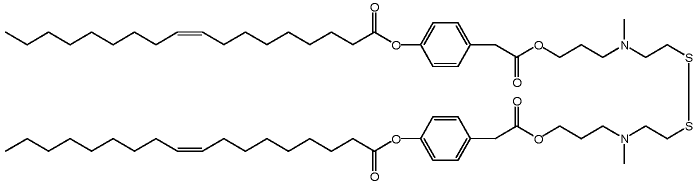 |
| TS-P4C2 (or SS-EC) | 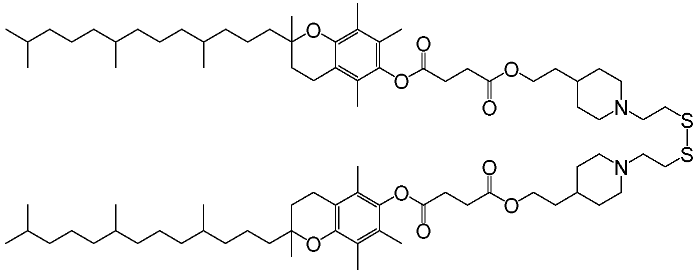 |

**[Table 1-3]**

| name of ionic lipid | structure |
|---|---|
| Lipid 1 | 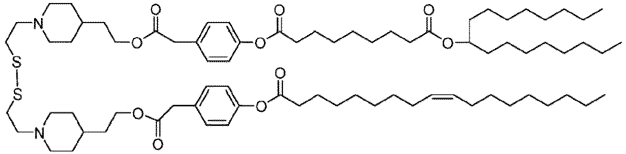 |
| Lipid 5 | 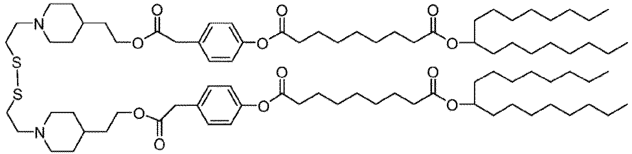 |
| Lipid 8 | 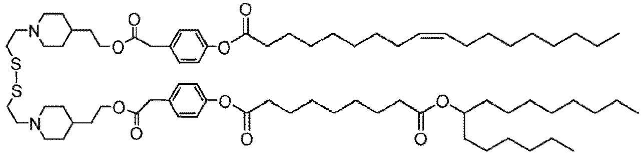 |

Among specific examples of ionic lipid (1), SS-OP is preferred. That is, ionic lipid (1) is preferably an ionic lipid represented by the following formula:

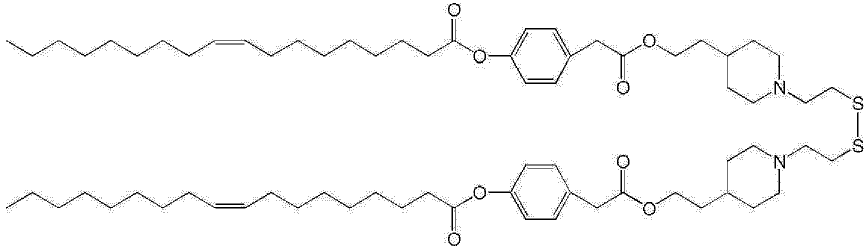

The amount of ionic lipid (1) in the lipid nanoparticles of the present invention is preferably 20 to 60 mol%, more preferably 25 to 55 mol%, further preferably 30 to 50 mol%, relative to the total amount of ionic lipid (1), phospholipid, and cholesterol, from the aspects of the nucleic acid encapsulation efficiency, the intracellular nucleic acid release efficiency, and the stability of the lipid nanoparticles.

Ionic lipid (1) can be produced by a known method (e.g., methods described in WO2019/188867A1, US9708628B2, WO2021/195529A2).

### Phospholipid

The lipid nanoparticles of the present invention contain, as a phospholipid, 1,2-diacyl-sn-glycero-3-phosphocholine (PC) in which the acyl groups have not less than 20 carbon atoms and at least one of the acyl groups is an alkenoyl group. Furthermore, only one type of the phospholipid (PC) may be used, or the (PC) or other phospholipids may be used in combination. In this case, the ratio of the other phospholipids to the (PC) is not particularly limited as long as it is within a range capable of exhibiting the effects of the present invention.

Specific examples the 1,2-diacyl-sn-glycero-3-phosphocholine in which the acyl groups have not less than 20 carbon atoms and at least one of the acyl groups is an alkenoyl group include 1-arachidoyl-2-eicosenoyl-sn-glycero-3-phosphocholine (AEiPC), 1-eicosenoyl-2-arachidoyl-sn-glycero-3-phosphocholine (EiAPC), 1,2-dieicosenoyl-sn-glycero-3-phosphocholine (DEiPC), 1-behenoyl-2-erucoyl-sn-glycero-3-phosphocholine (BEPC), 1-erucoyl-2-behenoyl-sn-glycero-3-phosphocholine (EBPC), 1,2-dierucoyl-sn-glycero-3-phosphocholine (DEPC), 1-lignoceroyl-2-nervonoyl-sn-glycero-3-phosphocholine (LiNPC), 1-nervonoyl-2-lignoceroyl-sn-glycero-3-phosphocholine (NLiPC), and 1,2-dinervonoyl-sn-glycero-3-phosphocholine (DNPC). In the present specification, phospholipids may be referred to by their abbreviations. For example, 1,2-diacyl-sn-glycero-3-phosphocholine may be referred to as PC, and 1,2-dierucoyl-sn-glycero-3-phosphocholine may be referred to as DEPC. Furthermore, using the carbon number and degree of unsaturation of the fatty acid, for example, 1,2-dierucoyl-sn-glycero-3-phosphocholine may be indicated as C22:1PC.

Phospholipid is preferably 1,2-diacyl-sn-glycero-3-phosphocholine in which the acyl groups have not less than 20 carbon atoms and at least one of the acyl groups has one unsaturated bond, more preferably 1,2-diacyl-sn-glycero-3-phosphocholine in which the acyl groups have not less than 20 carbon atoms and both acyl groups have one unsaturated bond, further preferably at least one selected from the group consisting of DEiPC, DEPC, and DNPC, most preferably DEPC. These phospholipids are considered to afford similar effects because they have similar acyl chain structures. The number of carbon atoms in the acyl group includes the carbonyl carbon. The number of carbon atoms in the acyl group is preferably 20 to 26, more preferably 20 to 24.

The amount of phospholipid in the lipid nanoparticles of the present invention is preferably 5 to 20 mol%, more preferably 10 to 20 mol%, further preferably 10 to 15 mol%, relative to the total of ionic lipid (1), phospholipid, and cholesterol, from the aspects of nucleic acid encapsulation efficiency, intracellular nucleic acid release efficiency, and lipid nanoparticle stability.

### Cholesterol

The lipid nanoparticles of the present invention contain cholesterol. The amount of cholesterol in the lipid nanoparticles of the present invention is preferably 30 to 70 mol%, more preferably 35 to 65 mol%, further preferably 40 to 60 mol%, relative to the total of ionic lipid (1), phospholipid, and cholesterol, from the aspects of nucleic acid encapsulation efficiency, intracellular nucleic acid release efficiency, and lipid nanoparticle stability.

### Dimyristoylglycerol PEG

The lipid nanoparticles of the present invention contains a dimyristoylglycerol PEG represented by the formula (2):

CH₂(OR⁶)-CH(OR⁷)-CH₂(OR⁸) (2)

(in the formula (2),
any two of R⁶, R⁷ and R⁸ are myristoyl groups, and the remaining one is an alkyl group having 1 - 6 carbon atoms connected via a polyethylene glycol (PEG) chain having a number average molecular weight of 1,000 to 3,000) (sometimes to be abbreviated as "dimyristoylglycerol PEG (2)" in the present specification).

The number average molecular weight of the PEG chain in the formula (2) is 1,000 to 3,000, preferably 1,500 to 2,500. The number average molecular weight of the PEG used to form this PEG chain can be measured by gel permeation chromatography (GPC) .

The alkyl group having 1 - 6 carbon atoms may be linear, branched or cyclic. The alkyl group preferably has a carbon number of 1 - 3. Specific examples of the alkyl group having 1 - 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like. It is preferably a methyl group.

The amount of dimyristoylglycerol PEG (2) in the lipid nanoparticles of the present invention is preferably 0.25 to 2 mol %, more preferably 0.5 to 1.5 mol %, further preferably 0.5 to 1.0 mol %, relative to the total amount of ionic lipid (1), phospholipid, and cholesterol, from the aspects of nucleic acid encapsulation efficiency, intracellular nucleic acid release efficiency, and lipid nanoparticle stability.

### Optimal composition

The optimal molar ratio of ionic lipid (1):phospholipid:cholesterol:dimyristoylglycerol PEG (2) in the lipid nanoparticles of the present invention is 40:10:50:0.75.

### Production method of lipid nanoparticles

The lipid nanoparticles of the present invention can be produced by dispersing lipid raw materials containing ionic lipid (1), phospholipid, cholesterol, and dimyristoylglycerol PEG (2) in an appropriate dispersion medium (e.g., aqueous dispersion medium, alcoholic dispersion medium), and performing an operation to induce organization as necessary.

Examples of the "operation to induce organization" for producing the lipid nanoparticles of the present invention include methods known per se such as an ethanol dilution method using a micro flow path or vortex, a simple hydration method, sonication, heating, vortex, an ether injecting method, a French press method, a cholic acid method, a Ca²⁺ fusion method, a freeze-thaw method, a reversed-phase evaporation method and the like, preferably an ethanol dilution method using a micro flow path or vortex, further preferably an ethanol dilution method using a micro flow path. In the ethanol dilution method using a micro flow path, for example, NanoAssemblr (registered trademark) (Precision NanoSystems) can be used to produce a dispersion containing lipid nanoparticles by mixing an acidic buffer containing nucleic acid with an ethanol solution of lipids. The dispersion produced by this method contains lipid nanoparticles and a dispersion medium (acidic buffer and ethanol), but removal of the dispersion medium (especially ethanol), exchange of the dispersion medium (particularly buffer), and the like can be performed by operations such as ultrafiltration, dialysis, dilution, and the like.

### Method for delivering nucleic acid to peripheral blood mononuclear cell

The present invention also provides a method for delivering nucleic acids to peripheral blood mononuclear cells including contacting the lipid nanoparticles encapsulating nucleic acid of the present invention with the target peripheral blood mononuclear cells. In this method, lipid nanoparticles encapsulating the nucleic acid are preferably transfected into the target cells.

Examples of the nucleic acid include, but are not limited to, DNA, RNA, chimera nucleic acid of RNA, DNA/RNA hybrid and the like. While any nucleic acid having 1 to 3 chains can be used, it is preferably a single strand or double strand. The nucleic acid may be nucleotide having N-glycoside of purine or pyrimidine base or oligomer having a non-nucleotide backbone (e.g., commercially available peptide nucleic acid (PNA) etc.), oligomer containing a special bond (said oligomer comprising base pairing or a nucleotide having a configuration permitting attachment of base, which are found in DNA and RNA) and the like.

Furthermore, it may be a nucleic acid added with known modification, for example, a nucleic acid with a label known in the field, a nucleic acid with a cap, a methylated nucleic acid, one or more natural nucleotides substituted by an analog, a nucleic acid with nucleotidyl modification, for example, a nucleic acid with non-charge bond (e.g., methylphosphonate, phosphotriester, phosphoramidate, carbamate and the like), a nucleic acid with a charged bond or sulfur-containing bond (e.g., phosphorothioate, phosphorodithioate and the like), for example, a nucleic acid with a side chain group such as protein (nuclease, nuclease inhibitor, toxin, antibody, signal peptide, poly-L-lysine and the like), sugar (e.g., monosaccharide and the like) and the like, a nucleic acid with an intercalating compound (e.g., acridine, psoralen and the like), a nucleic acid with a chelate compound (e.g., metal, radioactive metal, boron, oxidative metal and the like), a nucleic acid containing an alkylating agent, or a nucleic acid with a modified bond (e.g., α anomer-type nucleic acid and the like).

The type of the DNA that can be used in the present invention is not particularly limited, and can be selected as appropriate according to the purpose of use. As DNA, for example, plasmid DNA, cDNA, antisense DNA, chromosomal DNA, PAC, BAC, CpG oligosaccharide, and the like can be mentioned. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA that can be used in the present invention is not particularly limited, and can be selected as appropriate according to the purpose of use. As RNA, for example, siRNA, miRNA, shRNA, antisense RNA, messenger RNA (mRNA), single strand RNA genome, double strand RNA genome, RNA replicon, transfer RNA, ribosomal RNA and the like can be mentioned, with preference given to siRNA, miRNA, shRNA, mRNA, antisense RNA, and RNA replicon.

The nucleic acid used in the present invention is preferably purified by a method generally used by those of ordinary skill in the art.

The nucleic acid used in the present invention is a nucleic acid used for in vitro/ex vivo gene transfer, and is specifically a nucleic acid for gene transfer into primary cells extracted from a living organism for use in CAR-T therapy and the like. More specifically, the nucleic acid of the present invention is preferably a nucleic acid having prophylactic and/or therapeutic activity for patients with a certain disease (so-called prophylactic/therapeutic nucleic acid). Examples of such nucleic acid include nucleic acid and the like used for so-called gene therapy and cell therapy.

Examples of nucleic acid used in cell therapy include nucleic acids encoding chimeric antigen receptors (CARs) and T cell receptors (TCRs).

The nucleic acid encoding the CAR used in cell therapy includes an antigen-binding domain, an extracellular hinge domain, a transmembrane domain, and an intracellular T cell signaling domain, of an antibody capable of specifically recognizing a surface antigen to be recognized by a target immune cell.

The nucleic acid encoding the TCR used in cell therapy is a nucleic acid encoding the α chain and β chain of a TCR capable of specifically recognizing a surface antigen to be recognized by a target T cell.

The types of nucleic acids encoding CAR and TCR include, but are not limited to, DNA, RNA, RNA chimeric nucleic acid, DNA/RNA hybrid, and the like.

The particle size of the lipid nanoparticles encapsulating the nucleic acid is not particularly limited, and is preferably 10 nm - 500 nm, more preferably 30 nm - 300 nm. The particle size can be measured by using a particle size distribution measuring device such as Zetasizer Nano (Malvern) or the like. The particle size of the lipid nanoparticles encapsulating the nucleic acid can be appropriately adjusted by the production method thereof.

The surface potential (zeta potential) of the lipid nanoparticles encapsulating the nucleic acid is not particularly limited and preferably -15 to +15 mV, more preferably -10 to +10 mV. In conventional transgene, particles with positively charged surface have been mainly used. This is useful as a method for promoting electrostatic interactions with heparin sulfate on the negatively-charged cell surface to enhance uptake into cells. However, a positive surface potential can result in (a) inhibition of release of nucleic acid from the carrier through interaction with the delivered nucleic acid within cells, and (b) inhibition of protein synthesis through interaction between mRNA and the delivered nucleic acid. This problem can be solved by adjusting the surface potential (zeta potential) to fall within the above-mentioned range. The surface potential (zeta potential) can be measured using a zeta potential measuring apparatus such as Zetasizer Nano and the like. The surface potential (zeta potential) of the lipid nanoparticles can be adjusted by the composition of the constituent component of the lipid nanoparticles.

By contacting the lipid nanoparticles of the present invention encapsulating nucleic acid with peripheral blood mononuclear cells, the lipid nanoparticles are introduced into the peripheral blood mononuclear cells, and the nucleic acid encapsulated in the lipid nanoparticles is delivered to the peripheral blood mononuclear cells. Peripheral blood mononuclear cell is a general term for mononuclear cells isolated from peripheral blood, and the peripheral blood mononuclear cells into which the lipid nanoparticles are introduced are not particularly limited as long as they are mononuclear cells contained in peripheral blood. Examples of such cells include T cell, B cell, NK cell, dendritic cells, macrophage, monocytes, and the like. The organisms of the target cells are also not particularly limited, and examples of such cells include cells of mammals (e.g., humans, monkeys, mice, rats, hamsters, cows, etc.), birds (e.g., chickens, ostriches, etc.), amphibians (e.g., frogs, etc.), and fish (e.g., zebrafish, rice-fish, etc.). The subject into which the lipid nanoparticles are introduced is preferably a human or other mammalian cell.

The method for introducing the lipid nanoparticles encapsulating nucleic acid into the target cells is not particularly limited as long as the lipid nanoparticles can deliver the nucleic acid into the cells, and any method known per se (e.g., transfection, reverse transfection, etc.) can be appropriately selected. Transfection is preferred as a method for introducing nucleic acid into the target cells using the lipid nanoparticles of the present invention. The amount of the lipid nanoparticles to be used can be appropriately selected taking into consideration the type of the target cell, the introduction method, and the like.

The step of contacting lipid nanoparticles encapsulating nucleic acid with peripheral blood mononuclear cells in vitro (in vitro/ex vivo) is described in detail below.

Peripheral blood mononuclear cells are suspended in a suitable medium several days before contact with the lipid nanoparticles, and cultured under appropriate conditions. During the culture, an activating factor (e.g., anti-CD3 antibody/anti-CD28 antibody and the like in the case of T cells) or a growth factor (e.g., IL-2 and the like) may or may not be added. At the time of contact with the lipid nanoparticles, the cells may or may not be in a proliferative phase.

The culture medium on contact may be a serum-containing medium or a serum-free medium, wherein the serum concentration of the medium is preferably not more than 30 wt%, more preferably not more than 20 wt%, since when the medium contains excess protein such as serum and the like, the contact between the lipid nanoparticles and the cell may be inhibited.

The cell density on contact is not particularly limited, and can be appropriately determined in consideration of the kind of the peripheral blood mononuclear cell and the like. It is generally within the range of 1×10⁴ - 1×10⁷ cells/mL.

A suspension of the aforementioned lipid nanoparticles encapsulating the nucleic acid is added to the thus-prepared cells. The amount of the suspension to be added is not particularly limited, and can be appropriately determined in consideration of the cell number and the like. The concentration of the lipid nanoparticles to be contacted with the cells is not particularly limited as long as the desired introduction of the nucleic acid into the cells can be achieved. The lipid concentration is generally 1 - 300 nmol/mL, preferably 10 - 200 nmol/mL, and the concentration of the nucleic acid is generally 0.01 - 100 µg/mL, preferably 0.05 - 10 µg/mL.

After the aforementioned suspension is added to peripheral blood mononuclear cells, the cells are cultured. The temperature, humidity and CO₂ concentration during culturing are appropriately determined in consideration of the kind of the peripheral blood mononuclear cell. When the cell is derived from a mammal, generally, the temperature is about 37°C, humidity is about 95% and CO₂ concentration is about 5%. While the culture period can also be appropriately determined in consideration of the conditions such as the kind of the peripheral blood mononuclear cell and the like, it is generally a range of 0.1 to 120 hr, preferably a range of 0.2 to 96 hr, more preferably a range of 0.5 to 72 hr. When the above-mentioned culture time is too short, the nucleic acid is not sufficiently introduced into the cells, and when the culture time is too long, the cells may become weak.

By the above-mentioned culture, the nucleic acid is introduced into peripheral blood mononuclear cells. The culture is further continued preferably by exchanging the medium with a fresh medium, or adding a fresh medium to the medium. When the cell is a mammal-derived cell, the fresh medium preferably contains a serum or nutrition factor.

The lipid nanoparticles of the present invention can be produced as is or by mixing with a pharmaceutically acceptable carrier.

As the pharmaceutically acceptable carrier, those conventionally used as pharmaceutical materials are used, such as excipient, lubricant, solvent, solubilizer, suspending agent, isotonicity agent, buffer, and the like. Furthermore, formulation additives such as antiseptic, antioxidant, colorant, and sweeteners can also be used as necessary.

### [Example]

The Examples of the present invention are explained in further detail in the following, but the present invention is not limited by the following Examples.

In the following Examples, the ionic lipids represented by the formula (1) are shown by the names listed in the aforementioned Tables. The abbreviations used in the following Examples mean the following.
Chol: cholesterol
DEPC: 1,2-dierucoyl-sn-glycero-3-phosphocholine
DOPC: 1,2-dioleoyl-sn-glycero-3-phosphocholine
POPC: 1-palmitoyl-2-oleoyl-sn-glycero-3-phosphocholine
POPE: 1-palmitoyl-2-oleoyl-sn-glycero-3-phosphoethanolamine
DiD: 1,1'-dioctadecyl-3,3,3',3'-tetramethylindodicarbocyanine, 4-chlorobenzenesulfonate
DMG-PEG2000: 1,2-dimyristoyl-rac-glycerol, methoxypolyethylene glycol (number average molecular weight (Mn) of PEG: 2000)
MES: 2-morpholinoethanesulfonic acid
PBS: phosphate buffered saline
DDW: deionized distilled water

The mol% of the lipid composition indicates mol% relative to the total of ionic lipid (1), phospholipid, and cholesterol.

### [Production Example 1] Preparation of mRNA-encapsulated LNP

### (1) Preparation of lipid ethanol solution

The lipid ethanol solution was prepared by mixing 10 mM SS-OP, 5 mM phospholipid, and 10 mM Chol to a total concentration of 800 nmol, adding 1 mM DMG-PEG2000 ethanol solution to give the desired ratio, and adding ethanol to make the total volume 360 µL.

### (2) Preparation of acidic buffer solution of nucleic acid

The acidic buffer solution of nucleic acid was prepared by mixing mRNA encoding the luciferase gene with 20 mM acidic malic acid buffer (pH 3.0) containing 30 mM NaCl to a concentration of 0.0067 µg/µL.

### (3) Preparation of LNP by ethanol dilution method

Using a NanoAssmblr (registered trademark) ultrafast nanomedicine production device (manufactured by Precision NanoSystems), 1000 µL of acidic buffer solution of nucleic acid and 360 µL of lipid ethanol solution were mixed at flow rates of 3 mL/min and 1 mL/min, respectively, to obtain 0.8 mL of LNP solution. The obtained LNP was diluted with 3 mL of 20 mM MES buffer (pH 6.5) and transferred to Amicon Ultra 4 (Millipore). The transferred LNP solution was ultrafiltered under centrifugation conditions (25°C, 1000 g, about 10 min) to concentrate same to about 500 µL. The obtained concentrate was diluted to 4 mL with PBS, and concentrated again to about 250 µL by ultrafiltration under centrifugal conditions (25°C, 1000 g, about 10 min). Finally, the volume was increased with PBS to a nucleic acid concentration of 10 µg/mL.

### [Experimental Example 1] Lipid composition of various mRNA-encapsulated LNPs

Using the method described in Production Example 1, mRNA-encapsulated LNPs with the lipid composition shown in Table 2 below were prepared.

**[Table 2]**

| | lipid composition |
|---|---|
| Example 1 | SS-OP/DEPC/Chol/DMG-PEG2000=50/15/35/0.75 |
| Example 2 | SS-OP/DEPC/Chol/DMG-PEG2000=50/15/35/0.75 + DiD 0.2 |
| Example 3 | SS-OP/DEPC/Chol/DMG-PEG2000=60/5/35/0.75 |
| Example 4 | SS-OP/DEPC/Chol/DMG-PEG2000=55/10/35/0.75 |
| Example 5 | SS-OP/DEPC/Chol/DMG-PEG2000=45/20/35/0.75 |
| Example 6 | SS-OP/DEPC/Chol/DMG-PEG2000=40/10/50/0.75 |
| Example 7 | SS-OP/DEPC/Chol/DMG-PEG2000=35/15/50/0.75 |
| Example 8 | SS-OP/DEPC/Chol/DMG-PEG2000=30/20/50/0.75 |
| Example 9 | SS-OP/DEPC/Chol/DMG-PEG2000=60/10/30/0.75 |
| Example 10 | SS-OP/DEPC/Chol/DMG-PEG2000=50/10/40/0.75 |
| Example 11 | SS-OP/DEPC/Chol/DMG-PEG2000=45/15/40/0.75 |
| Example 12 | SS-OP/DEPC/Chol/DMG-PEG2000=30/10/60/0.75 |
| Example 13 | SS-OP/DEPC/Chol/DMG-PEG2000=25/15/60/0.75 |
| Example 14 | SS-OP/DEPC/Chol/DMG-PEG2000=20/10/70/0.75 |
| Example 15 | SS-OP/DEPC/Chol/DMG-PEG2000=40/10/50/0.5 |
| Example 16 | SS-OP/DEPC/Chol/DMG-PEG2000=40/10/50/1 |
| Example 17 | SS-OP/DEPC/Chol/DMG-PEG2000=40/10/50/1.25 |
| Example 18 | SS-OP/DEPC/Chol/DMG-PEG2000=40/10/50/1.5 |
| Comparative Example 1 | SS-OP/POPC/Chol/DMG-PEG2000=50/15/35/0.75 |
| Comparative Example 2 | SS-OP/POPE/Chol/DMG-PEG2000=50/15/35/0.75 |
| Comparative Example 3 | SS-OP/POPC/Chol/DMG-PEG2000=50/15/35/0.75 + DiD 0.2 |
| Comparative Example 4 | SS-OP/POPE/Chol/DMG-PEG2000=50/15/35/0.75 + DiD 0.2 |
| Comparative Example 5 | SS-OP/DOPC/Chol/DMG-PEG2000=50/15/35/0.75 |
| Comparative Example 6 | SS-OP/POPE/Chol/DMG-PEG2000=40/10/50/0.75 |

(In Table, the composition ratio is in mol%)

### [Experimental Example 2] Evaluation of gene expression activity in mouse primary T cells

### (1) Isolation of mouse primary T cells

After incising the abdominal cavity of a C57BL/6JJcl mouse (Japan SLC), the spleen was collected, and the spleen was crushed by rubbing microscope slide glasses on PBS to separate the spleen cells. The cell suspension was passed through a 100 um filter (MACS SmartStrainers (100 µm); Miltenyi Biotec) and a 30 um filter (MACS SmartStrainers (30 µm); Miltenyi Biotec), and the cell pellet was made under centrifugation conditions (4°C, 400 g, 3 min). The supernatant was removed, and the cells were suspended in 90 µL of MACS Buffer (0.5% BSA, 2 mM EDTA in PBS), 10 µL of CD90.2 MicroBeads (Miltenyi Biotec) were added, and the cells were mixed by gentle tapping and left on ice for 15 min. Then, 5 mL of MACS Buffer was added and the cell pellet was made under centrifugation conditions (4°C, 400 g, 3 min). The supernatant was removed, and the cells were suspended in 500 µL of degassed MACS buffer. 500 µL of degassed MACS buffer was passed through a column (MS Columns; Miltenyi Biotec) in contact with a magnet, and the column was left standing until no liquid fell, after which a cell suspension suspended in degassed MACS buffer was added to the column and the mixture was left standing. 500 µL of degassed MACS buffer was passed through the column, followed by being left standing, and this process was repeated two more times. The column was removed from the magnet, 1 mL of degassed MACS buffer was added, and the suspension was pushed out with a piston to recover mouse primary T cells. The cell pellet was made under centrifugation conditions (4°C, 400 g, 3 min), the supernatant was removed, and the cells were suspended in 1 mL of RPMI1640 (10% FBS, 1 mM pyruvate, 10 mM HEPES, 100 U/mL penicillin, 100 mg/mL streptomycin, 4.5 g/L glucose, 55 µM 2-mercaptoethanol, non-essential amino acids).

### (2) Culture of mouse primary T cells

2 mL of PBS was added to a 6-well plate (ThermoFisher), and anti-CD3 antibody (Ultra-LEAF^{™} Purified anti-mouse CD3ε Antibody; BioLegend) and anti-CD28 antibody (Ultra-LEAF^{™} Purified anti-mouse CD28 Antibody; BioLegend) were added at 10 µg/mL and 0.5 µg/mL, respectively, and the plate was left standing for 3 hr. The supernatant was then removed, and 2 mL of PBS was added. The same procedure was repeated once more, and after removing the supernatant, 2 mL of mouse primary T cells isolated in (1) were seeded at a concentration of 1.5×10⁶ cells/mL, and 50 U/mL IL-2 (IL-2, Human Recombinant; PeproTech) was added. After 72 hr, 2 mL of medium containing 50 U/mL IL-2 was added. After further 48 hr, the cells were collected and used for various experiments.

### (3) Measurement of gene expression activity

Cultured mouse primary T cells were transfected with the LNPs of Example 1, Comparative Example 1, and Comparative Example 2 such that the amount of nucleic acid was 0.4 µg. Further, an aqueous D-luciferin potassium solution (prepared with DDW) was added such that the final luciferin concentration was 0.1 mM. After transfection, luminescence was measured for 100 hr using an incubating luminometer (Kronos Dio; ATTO), and the total luminescence amount was calculated by AUC as the gene expression amount. The results are shown in Fig. 1. The LNP of Example 1, in which DEPC was used as the phospholipid, improved gene expression activity in mouse primary T cells compared to Comparative Examples 1 and 2.

### [Experimental Example 3] Evaluation of LNP uptake into mouse primary T cells

### (1) Preparation of fluorescence-labeled LNP

A lipid ethanol solution containing a fluorescent dye was prepared by adding an ethanol solution of DiD to the lipid ethanol solution described in Production Example 1 such that the proportion was 0.2 mol% relative to the total lipid amount. This lipid ethanol solution was used to prepare LNP according to the procedure described in Production Example 1, to obtain fluorescence-labeled LNP.

### (2) Evaluation of LNP uptake into mouse primary T cells

Cultured mouse primary T cells were transfected with the LNPs of Example 2, Comparative Example 3, and Comparative Example 4, and the cells were collected after 24, 48, and 72 hr. The amount of LNP uptake was then evaluated using a flow cytometer. The results are shown in Fig. 2. The LNP of the composition of Example 2 using DEPC showed increased uptake into primary T cells compared to the LNPs of the compositions of Comparative Examples 3 and 4 at all time points.

### [Experimental Example 4] Effect of phospholipid composition ratio on gene expression activity in mouse primary T cells

Cultured mouse primary T cells were transfected with the LNPs of Examples 1 and 3 to 8, and Comparative Example 1, prepared in Production Example 1, such that the amount of nucleic acid was 0.4 µg. Further, an aqueous D-luciferin potassium solution was added such that the final luciferin concentration was 0.1 mM. After transfection, luminescence was measured for 100 hr using an incubating luminometer (Kronos Dio; ATTO), and the total luminescence amount was calculated by AUC as the gene expression amount. The results are shown in Fig. 3. The compositions of Examples 6 and 7, which used 10 or 15 mol% DEPC, showed high gene expression activity.

### [Experimental Example 5] Effect of cholesterol composition ratio on gene expression activity in mouse primary T cells

Cultured mouse primary T cells were transfected with the LNPs of Examples 6, 7, 9 to 14, and Comparative Example 1, prepared in Production Example 1, such that the amount of nucleic acid was 0.4 µg. Further, an aqueous D-luciferin potassium solution was added such that the final luciferin concentration was 0.1 mM. After transfection, luminescence was measured for 100 hr using an incubating luminometer (Kronos Dio; ATTO), and the total luminescence amount was calculated by AUC as the gene expression amount. The results are shown in Fig. 4. The composition of Example 6, which used 50 mol% cholesterol, showed the highest gene expression activity.

### [Experimental Example 6] Effect of DMG-PEG2000 composition ratio on gene expression activity in mouse primary T cells

Cultured mouse primary T cells were transfected with the LNPs of Examples 6, 15 to 18, and Comparative Example 1, prepared in Production Example 1, such that the amount of nucleic acid was 0.4 µg. Further, an aqueous D-luciferin potassium solution was added such that the final luciferin concentration was 0.1 mM. After transfection, luminescence was measured for 100 hr using an incubating luminometer (Kronos Dio; ATTO), and the total luminescence amount was calculated by AUC as the gene expression amount. The results are shown in Fig. 5. The composition of Example 6, which used 0.75 mol% DMG-PEG2000, showed the highest gene expression activity.

### [Experimental Example 7] Evaluation of gene expression activity in human primary T cells

### (1) Culture of human primary T cells

Human peripheral blood CD3+ Pan T cells (purchased from Lonza) were thawed at 37°C. After adding 9 mL of RPMI1640 (10% FBS, 1 mM pyruvate, 10 mM HEPES, 100 U/mL penicillin, 100 mg/mL streptomycin, 4.5 g/L glucose, 55 µM 2-mercaptoethanol, non-essential amino acid), the cells were centrifuged under centrifugation conditions (4°C, 300 g, 10 min). The supernatant was removed, the cells were suspended in 10 mL of medium, and 1 mL of the cells were seeded in a 12-well plate (ThermoFisher) at a concentration of 1×106 cells/mL. Dynabeads Human T-Activator CD3/CD28 (ThermoFisher) were added such that the beads:T cell ratio was 1:2, and 30 U/mL IL-2 (PeproTech) was added. After 72 hr, the mixture was split such that the cell concentration was halved. 24 hr later, 1 mL of medium containing 30 U/mL IL-2 was added. After 72 hr, the cells were collected, the Dynabeads were removed, and the cells were seeded at 2×10⁵ cells/mL and used in the experiment.

### (2) Measurement of gene expression activity

Cultured human primary T cells were transfected with the LNPs of Example 6, Comparative Example 5, and Comparative Example 6, prepared in Production Example 1, such that the amount of nucleic acid was 0.4 µg. Further, an aqueous D-luciferin potassium solution was added such that the final luciferin concentration was 0.1 mM. After transfection, luminescence was measured for 40 hr using an incubating luminometer (Kronos Dio; ATTO), and the total luminescence amount was calculated by AUC as the gene expression amount. The results are shown in Fig. 6. Also in human primary T cells, the LNP having the composition of Example 6 using DEPC showed higher gene expression activity than Comparative Example 5 and Comparative Example 6.

### [Industrial Applicability]

The lipid nanoparticle of the present invention is useful for delivering nucleic acids to peripheral blood mononuclear cells.

This application is based on patent application No. 2022-051919 filed in Japan, the contents of which are encompassed in full herein.

## Claims

1. A lipid nanoparticle for use in delivering a nucleic acid to a peripheral blood mononuclear cell, comprising an ionic lipid represented by the formula (1): 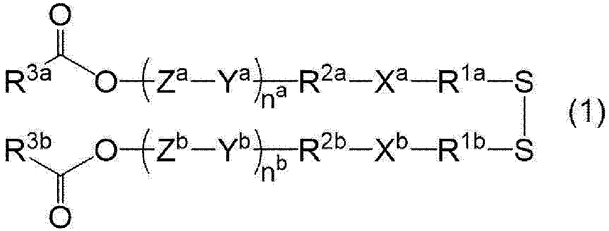 (in the formula (1),
R^{1a} and R^{1b} are each independently an alkylene group having 1 - 6 carbon atoms,
X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 - 16 carbon atoms and at least one aromatic ring, and optionally having a hetero atom,
n^{a} and n^{b} are each independently 0 or 1, and
R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):
R⁹-O-CO-(CH₂)a- (4)
(in the formula (4),
R⁹ is an aliphatic hydrocarbon group having 2 - 20 carbon atoms, and
a is an integer of 2 to 10)),
a phospholipid that is 1,2-diacyl-sn-glycero-3-phosphocholine in which the acyl groups have not less than 20 carbon atoms and
at least one of the acyl groups is an alkenoyl group, cholesterol, and
a dimyristoylglycerol PEG represented by the formula (2):
CH₂(OR⁶)-CH(OR⁷)-CH₂(OR⁸) (2)
(in the formula (2),
any two of R⁶, R⁷ and R⁸ are myristoyl groups, and the remaining one is an alkyl group having 1 - 6 carbon atoms connected via a polyethylene glycol chain having a number average molecular weight of 1,000 to 3,000).

2. The lipid nanoparticle according to claim 1, wherein the phospholipid is at least one selected from the group consisting of 1,2-dieicosenoyl-sn-glycero-3-phosphocholine (DEiPC), 1,2-dierucoyl-sn-glycero-3-phosphocholine (DEPC), and 1,2-dinervonoyl-sn-glycero-3-phosphocholine (DNPC).

3. The lipid nanoparticle according to claim 1 or 2, wherein the ionic lipid represented by the formula (1) is an ionic lipid represented by the following formula:

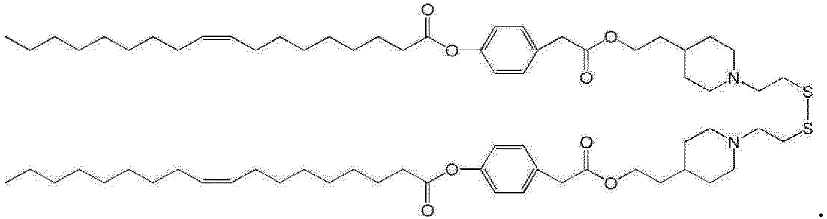

4. The lipid nanoparticle according to any one of claims 1 to 3, wherein the ionic lipid is 20 to 60 mol%, the phospholipid is 5 to 20 mol%, the cholesterol is 30 to 70 mol%, and the dimyristoylglycerol PEG lipid is 0.5 to 1.5 mol% relative to the total of the ionic lipid, the phospholipid and the cholesterol.

5. A method for delivering a nucleic acid to a peripheral blood mononuclear cell, comprising contacting the lipid nanoparticle according to any one of claims 1 to 4 encapsulating the nucleic acid with the peripheral blood mononuclear cell.

6. Use of a lipid nanoparticle comprising an ionic lipid represented by the formula (1): 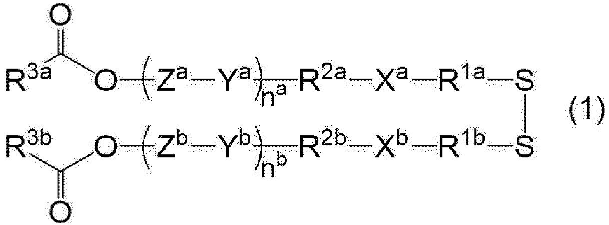 (in the formula (1),
R^{1a} and R^{1b} are each independently an alkylene group having 1 - 6 carbon atoms,
X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 - 16 carbon atoms and at least one aromatic ring, and optionally having a hetero atom,
n^{a} and n^{b} are each independently 0 or 1, and
R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (4):
R⁹-O-CO-(CH₂)a- (4)
(in the formula (4),
R⁹ is an aliphatic hydrocarbon group having 2 - 20 carbon atoms, and
a is an integer of 2 to 10)),
a phospholipid that is 1,2-diacyl-sn-glycero-3-phosphocholine in which the acyl groups have not less than 20 carbon atoms and
at least one of the acyl groups is an alkenoyl group, cholesterol, and
a dimyristoylglycerol PEG represented by the formula (2):
CH₂(OR⁶)-CH(OR⁷)-CH₂(OR⁸) (2)
(in the formula (2),
any two of R⁶, R⁷ and R⁸ are myristoyl groups, and the remaining one is an alkyl group having 1 - 6 carbon atoms connected via a polyethylene glycol chain having a number average molecular weight of 1,000 to 3,000),
in the production of a medicament for delivering a nucleic acid to a peripheral blood mononuclear cell.
